Europäisches Patentamt

European Patent Office

Office européen des brevets

(19)

(11) Veröffentlichungsnummer: **0 024 701**
**B1**

(12) # EUROPÄISCHE PATENTSCHRIFT

(45) Veröffentlichungstag der Patentschrift:
**21.09.83**

(21) Anmeldenummer: **80104998.2**

(22) Anmeldetag: **22.08.80**

(51) Int. Cl.³: **B 01 D 19/00,** B 01 J 10/00,
C 12 M 1/08

(54) Verfahren zur Verbesserung der Gasabscheidung in Flüssigkeits-/Gas-Reaktoren.

(30) Priorität: 04.09.79 DE 2935639

(43) Veröffentlichungstag der Anmeldung:
**11.03.81 Patentblatt 81/10**

(45) Bekanntmachung des Hinweises auf die Patenterteilung:
**21.09.83 Patentblatt 83/38**

(84) Benannte Vertragsstaaten:
**AT BE CH DE FR GB IT LI NL SE**

(56) Entgegenhaltungen:
**DE-A-1 805 360**
**FR-A-2 274 346**
**GB-A-1 491 502**
**US-A-2 518 845**
**US-A-3 377 778**
**US-A-3 626 670**

**Handbook of Chemistry and Physics 55th Ed C-769**

(73) Patentinhaber: **HOECHST AKTIENGESELLSCHAFT,**
**Postfach 80 03 20, D-6230 Frankfurt am Main 80 (DE)**

(72) Erfinder: **Ramspeck, Wolfgang, Taunusstrasse 60,**
**D-6370 Oberursel/Taunus (DE)**
Erfinder: **Sittig, Wolfgang, Sulzbacher Weg 2,**
**D-6238 Hofheim am Taunus (DE)**

## Verfahren zur Verbesserung der Gasabscheidung in Flüssigkeits-/Gas-Reaktoren

Zur Abscheidung der Gasphase in Flüssigkeits-/Gas-Reaktoren werden üblicherweise Zyklone, Schaumabscheider, Demister-ähnliche Einrichtungen und andere eingesetzt, die bei grossem Volumen grosse Oberfläche garantieren. Solche Flüssigkeits-/Gas-Reaktoren werden zur Vergrösserung der Phasengrenzfläche oft mit einer Füllkörperschicht versehen und/oder als Fliessbettreaktoren betrieben.

Aus Untersuchungen mit solchen Reaktoren sind phänomenologische Beschreibungen von Blasengrössen-Änderungen in Fliessbetten und/oder Füllkörperschichten bekannt.

Aufgabe der Erfindung war es, ein Verfahren zur Verbesserung der Gasabscheidung in Flüssigkeits-/Gas-Reaktoren zu entwickeln.

Gefunden wurde ein Verfahren zur Verbesserung der Koaleszenz und Abscheidung von feinen Gasblasen in der Entgasungszone von Flüssigkeits-/Gas-Reaktoren, das dadurch gekennzeichnet ist, dass der Flüssigkeits-/Gas-Strom in eine als Wirbelschicht fungierende Schüttung aus (gegenüber dem Flüssigkeits-/Gas-Gemisch) spezifisch schwereren Feststoff-Körpern eingeleitet wird, die eine Dichte von 0,5 bis 13 g/cm³ und ein Nennmass von 0,1 bis 100 mm aufweisen, wobei die Feststoff-Körper in Grösse, Dichte und Form so ausgewählt werden, dass ihre Sinkgeschwindigkeit grösser ist als die auf den freien Reaktorquerschnitt bezogene Geschwindigkeit des Flüssigkeits-/Gas-Gemisches (kurz Flüssigkeitsgeschwindigkeit), und dass anderseits die zu ihrer Fluidisierung notwendige Wirbelpunktsgeschwindigkeit kleiner ist als die genannte Flüssigkeitsgeschwindigkeit.

Zweckmässigerweise wird die Feststoffkörper-Schicht auf einem Träger angeordnet, der beispielsweise als Siebboden oder Auflagerost oder Drahtgewebematte ausgebildet sein kann.

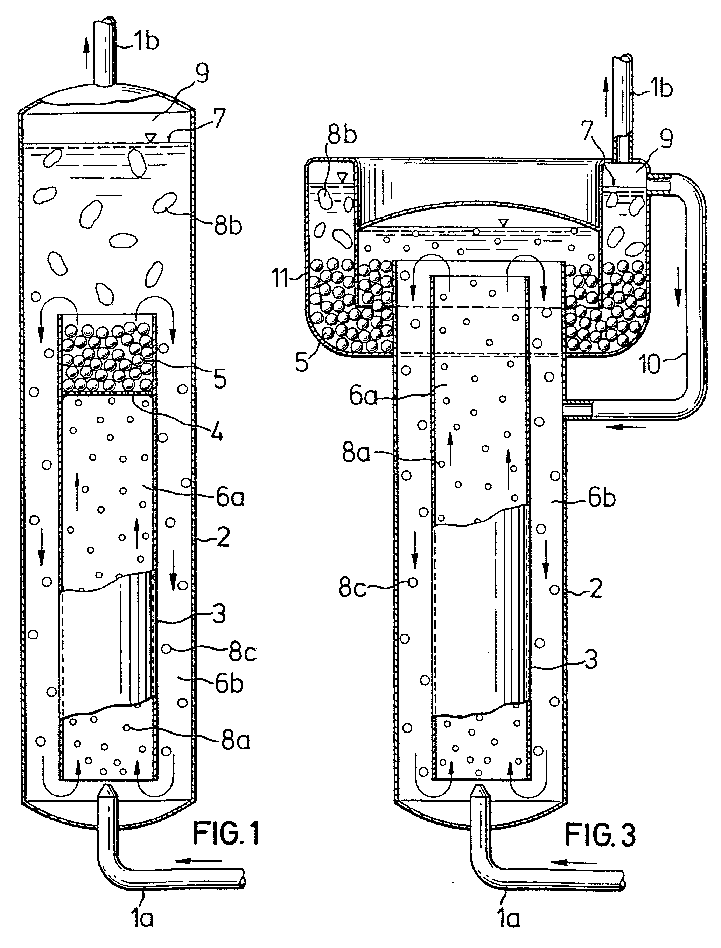

Beispiele für erfindungsgemäss zur verbesserten Gasabscheidung angeordnete Feststoffkörper-Schichten sind in den Fig. 1 bis 4 dargestellt. Darin bedeuten:

1a Gaseintritt
1b Gasaustritt
2 Reaktor
3 Innenrohr
4 Träger
5 Feststoffkörperschicht
6a Aufstromteil (eines Schlaufenreaktors)
6b Abstromteil (eines Schlaufenreaktors)
6c Blasensäulenteil
7 Flüssigkeitsstand
8a nicht koaleszierte Gasbläschen
8b/c koaleszierte Gasblasen
9 Gasraum
10 Flüssigkeitsrückführung
11 Umlaufender Ringraum
12 Tauchrohr
13 Gasabscheider.

Fig. 1 stellt einen Schlaufenreaktor (2) dar. Das durch den Gaseintritt (1a) zugeführte Gas steigt im Aufstromteil (6a) im Innenrohr (3) in Form mehr oder weniger kleiner Bläschen (8a) – abhängig von der Gasart, der Flüssigkeitsart und der Strömungsgeschwindigkeit – empor. Im oberen Teil des Innenrohres (3) befindet sich auf einem Träger (4) (z.B. Siebboden oder Auflagerost) eine Feststoffkörperschicht (5). In dieser Feststoffkörperschicht findet eine verstärkte Koaleszenz der feinen Gasbläschen statt. Die koaleszierten Gasblasen (8b) treten in den Gasraum (9) über. Ein kleiner Teil von ihnen (8c), deren Aufstieggeschwindigkeit kleiner als die Flüssigkeitsgeschwindigkeit ist, werden im Abstromteil (6b) mitgeführt.

In Fig. 2 ist die erfindungsgemässe Anordnung der Festkörperschicht im Blasensäulen-Teil (6c) einer Kombination aus Schlaufenreaktor und aufgesetzter Blasensäule (6c) dargestellt.

Um bei hohen Gasbelastungen die Rückführung der Flüssigkeit durch die Feststoffkörperschicht sicherzustellen, kann eine externe Flüssigkeitsrückführung (10) vorgesehen werden.

In Fig. 3 ist eine weitere Variante dargestellt: Die Feststoffkörperschicht befindet sich hier in einem umlaufenden Ringraum (11) am oberen Ende eines Schlaufenreaktors.

In Fig. 4 schliesslich befindet sich die Feststoffkörperschicht in einem vom Schlaufenreaktor getrennten Gasabscheider (13), der durch ein Tauchrohr (12) mit diesem verbunden ist.

Der Gasabscheider (13) kann auch als zylindrischer Behälter (ohne die dargestellte Verjüngung) mit Flach-, Klöpper- oder Halbrundboden ausgebildet sein.

Prinzipiell kann das erfindungsgemässe Verfahren zur verbesserten Gasabscheidung statt in einem Schlaufenreaktor auch bei jedem anderen Zweiphasen-Reaktor angewendet werden, z.B. bei einem Blasensäulenreaktor oder Rührkesselreaktor oder Rohrreaktor. Die Feststoffkörperschicht ist hier jeweils in der Entgasungszone angeordnet.

Die Dichte der erfindungsgemäss einzusetzenden Feststoffkörper beträgt von 0,5 bis 13 g/cm³, in wässrigen Systemen vorzugsweise von 1,1 bis 4 g/cm³. Als Materialien eignen sich beispielsweise keramische Werkstoffe, Glas, Kunststoffe, Metalle und Metallegierungen. Zweckmässigerweise sollten die verwendeten Materialien keinen oder nur geringfügigen Abrieb aufweisen, der die Produktionsqualität nicht beeinträchtigt.

Als Form der Feststoffkörperteilchen können alle Formen der üblicherweise verwendeten Füllkörper gewählt werden. Vorzugsweise sind die Teilchen kugel- oder linsenförmig. Das Nennmass (bei Kugeln der Durchmesser) der Teilchen kann im Bereich von 0,1 bis 100 mm gewählt werden.

Für Form, Dichte und Grösse der Teilchen sind die oben angeführten Geschwindigkeitsverhältnisse im jeweils vorliegenden Flüssigkeits-/Gas-Gemisch massgebend.

Die Höhe der Wirbelschicht (Feststoffkörperschicht) beträgt vorzugsweise das 0,2- bis 3fache ihres grössten Durchmessers.

Bei leicht schäumenden Medien ist es vorteilhaft, im Bereich der Wirbelschicht ein Schaumbekämpfungsmittel einzubringen.

Das anmeldungsgemässe Verfahren eignet sich zur verbesserten Gasabscheidung aus Flüssigkeiten in Form von Lösungen, Suspensionen oder Emulsionen, beispielsweise Katalysatorsuspensionen oder Fermentationskultursuspensionen.

Beispiele

1) Vergleichsbeispiel

In einem Schlaufenreaktor (betrieben nach dem Prinzip Mammut-Pumpe) von 300 mm $\varnothing$, 4 m Höhe und 200 mm Innenrohr-$\varnothing$ mit aufgesetzter Blasensäule wurde eine Fermentationskultursuspension (Methylomonas clara) in einem Nährmedium gezüchtet, das folgende Zusammensetzung hatte:

| | | | | |
|---|---|---|---|---|
| $H_3PO_4$ | 85%ig | 1,9 | l | pro 1000 l Wasser |
| $NH_4OH$ | 25%ig | 2,8 | l | pro 1000 l Wasser |
| $K_2SO_4$ | | 1,4 | kg | pro 1000 l Wasser |
| $MgSO_4 \cdot 7H_2O$ | | 0,9 | kg | pro 1000 l Wasser |
| $Na_2SO_4$ | | 0,25 | kg | pro 1000 l Wasser |
| $CaCO_3$ | | 0,14 | kg | pro 1000 l Wasser |
| $Fe_2(SO_4)_3 \cdot 3H_2O$ | | 0,085 | kg | pro 1000 l Wasser |
| Spurenelementlösung | | 1,0 | l | pro 1000 l Wasser |

Der Gasgehalt (Luftgehalt) des Flüssigkeits-/Gas-Gemisches betrug 13%, der Sauerstoffeintrag 6,4 kg/m³h. Die stossende Oberfläche und die Höhe der emporgeschleuderten Flüssigkeitselemente zeigten an, dass die Gasbelastungsgrenze mit dem eingestellten Wert von 0,15 m/s Gas-Leerrohrgeschwindigkeit erreicht war. Die ungenügende Gasabscheidung liess eine Erhöhung der Gasbelastung wegen Schaumbildung nicht zu.

2) Erfindungsgemässes Beispiel

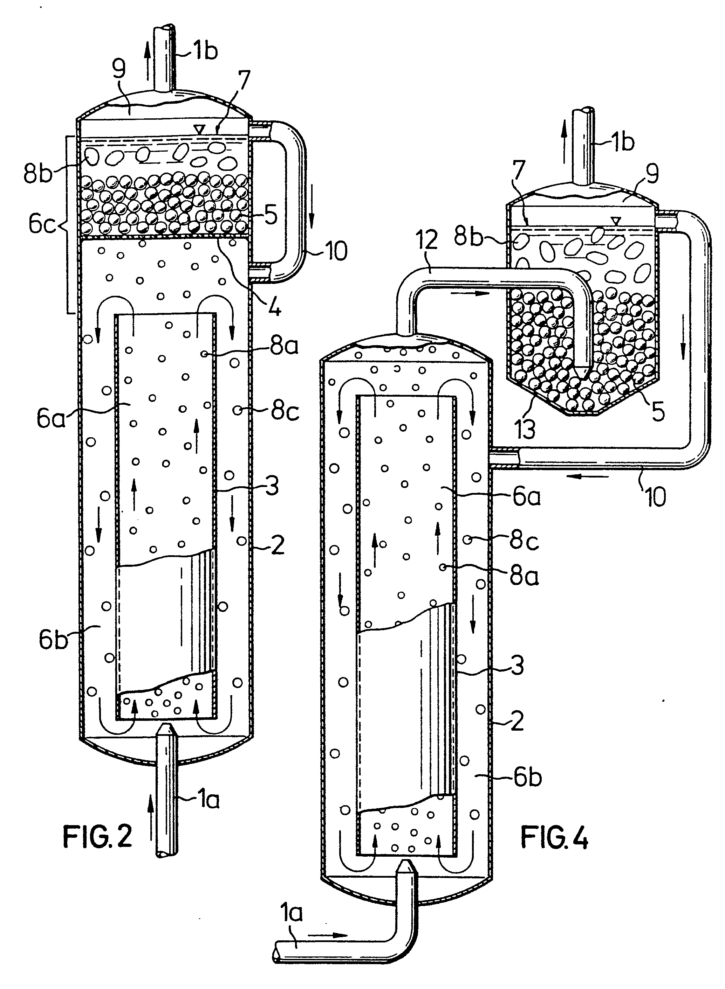

Es wurde im Blasensäulenteil der im Beispiel 1 verwendeten Kombination aus Schlaufenreaktor und aufgesetzter Blasensäule ein Siebboden installiert (wie in Fig. 2 dargestellt) mit einer Schicht von 150 mm Höhe (ruhend gemessen) aus Keramikkugeln mit einer Dichte von 2,7 g/cm³ und einem $\varnothing$ von 12 mm. Nährmedium, Gasgehalt und Abmessungen des Schlaufenreaktors waren gleich wie im Beispiel 1. Die Leerrohrgeschwindigkeit war 20% höher als im Beispiel 1. Der Sauerstoffeintrag betrug 7,8 kg/m³h.

**Patentansprüche**

1. Verfahren zur Verbesserung der Koaleszenz und Abscheidung von feinen Gasblasen in der Entgasungszone von Flüssigkeits-/Gas-Reaktoren, dadurch gekennzeichnet, dass der Flüssigkeits-/Gas-Strom in eine als Wirbelschicht fungierende Schüttung aus (gegenüber dem Flüssigkeits-/Gas-Gemisch) spezifisch schwereren Feststoff-Körpern eingeleitet wird, die eine Dichte von 0,5 bis 13 g/cm³ und ein Nennmass von 0,1 bis 100 mm aufweisen, wobei die Feststoff-Körper in Grösse, Dichte und Form so ausgewählt werden, dass ihre Sinkgeschwindigkeit grösser ist als die auf den freien Reaktorquerschnitt bezogene Geschwindigkeit des Flüssigkeits-/Gas-Gemisches (kurz Flüssigkeitsgeschwindigkeit), und dass anderseits die zu ihrer Fluidisierung notwendige Wirbelpunktgeschwindigkeit kleiner ist als die genannte Flüssigkeitsgeschwindigkeit.

2. Verfahren nach Anspruch 1, dadurch gekennzeichnet, dass die Höhe der Wirbelschicht das 0,2- bis 3fache ihres grössten Durchmessers beträgt.

3. Verfahren nach Ansprüchen 1 und 2, dadurch gekennzeichnet, dass die Wirbelschicht auf einem Träger im Aufstiegsteil eines Schlaufenreaktors oder in der Entgasungszone eines Blasensäulenreaktors, Rührkesselreaktors, Rohrreaktors oder anderen Zweiphasen-Reaktors angeordnet ist.

4. Verfahren nach Ansprüchen 1 und 2, dadurch gekennzeichnet, dass die Wirbelschicht in einem Gasabscheiderraum ausserhalb des eigentlichen Reaktors mit Flüssigkeitsrückführung angeordnet ist.

5. Verfahren nach Anspruch 4, dadurch gekennzeichnet, dass die Wirbelschicht in einem zylindrischen oder sich nach unten verjüngenden Abscheiderraum ohne Träger angeordnet ist, und dass das Flüssigkeits-/Gas-Gemsich mittels Tauchrohr in Bodennähe dieses Abscheiderraums eingeleitet wird.

6. Verfahren nach Ansprüchen 1 bis 5, dadurch gekennzeichnet, dass in die Wirbelschicht ein Schaumbekämpfungsmittel eingebracht wird.

**Claims**

1. A process for improving the coalescence and separation of fine gas bubbles in the degassing zone of liquid/gas reactors, wherein the liquid/gas current is introduced into a layer of solid particles acting as fluidized bed and having an increased specific weight (relative to the liquid/gas mixture), the density of which is from 0.5 to 13 g/cm³ and the nominal size of which is from 0.1 to 100 mm; the solid particles being chosen with respect to size, density and shape in such a manner that their sedimentation velocity is higher than the speed of the liquid/gas mixture relative to the free reactor cross-section (for short: liquid speed), and that on the other hand the fluidization point speed necessary for their fluidization is inferior to that of the cited liquid speed.

2. The process as claimed in Claim 1, wherein the height of the fluidized bed is 0.2 to 3 times that of its largest diameter.

3. The process as claimed in Claims 1 and 2, wherein the fluidized bed is arranged on a support in the upstream zone of a loop reactor, or in the degassing zone of a bubble-cap column reactor, agitator vessel reactor, tube reactor, or another two-phase reactor.

4. The process as claimed in Claims 1 and 2, wherein the fluidized bed is arranged in a gas separator outside the reactor with recycling equipment for the liquid.

5. The process as claimed in Claim 4, wherein the fluidized bed is arranged without support in a separator being cyclindric or tapered downward, and that the liquid/gas mixture is fed to this separator near its bottom via a dip pipe.

6. The process as claimed in Claims 1 to 5, wherein an anti-foaming agent is introduced into the fluidized bed.

**Revendications**

1. Procédé pour améliorer la coalescence et la séparation de petites bulles de gaz dans la zone de dégazage de réacteurs gaz-liquide, caractérisé en ce que l'on introduit le courant de gaz-liquide dans une masse, jouant le rôle d'un lit fluidisé, de corps solides de densité supérieure à celle du mélange gaz-liquide, présentant une masse volumique de 0,5 à 13 g/cm³ et une dimension nominale de 0,1 à 100 mm, la taille, la forme et la densité des corps solides étant choisies de façon que leur vitesse de descente soit supérieure à la vitesse, rapportée à la section transversale libre du réacteur, du mélange gaz-liquide (en abrégé, la vitesse du liquide), et que, par ailleurs, la vitesse du point de fluidisation nécessaire à la fluidisation soit inférieure à la vitesse du liquide indiquée ci-dessus.

2. Procédé selon la revendication 1, caractérisé en ce que la hauteur du lit fluidisé est égale à 0,2–3 fois son diamètre maximum.

3. Procédé selon l'une quelconque des revendications 1 ou 2, caractérisé en ce que le lit fluidisé est disposé sur un support (4) se trouvant dans la partie ascendante d'une colonne à écoulement en boucle ou dans la zone de dégazage d'un réacteur à colonne à barbotage, d'un réacteur avec agitateur, d'un réacteur tubulaire ou d'un autre réacteur biphasique.

4. Procédé selon l'une quelconque des revendications 1 ou 2, caractérisé en ce que le lit fluidisé est disposé dans une chambre de séparation des gaz à l'extérieur du réacteur proprement dit, avec une conduite de retour du liquide (10).

5. Procédé selon la revendication 4, caractérisé en ce que le lit fluidisé est disposé dans une chambre de séparation cylindrique ou présentant une conicité dirigée vers le bas, sans support, et que le mélange gaz-liquide est introduit au moyen d'un tube plongeur (12) au voisinage du fond de cette chambre de séparation.

6. Procédé selon l'une quelconque des revendications 1 à 5, caractérisé en ce qu'un produit antimoussant est introduit dans le lit fluidisé.

FIG.1

FIG.3

FIG. 2

FIG. 4